# EUROPEAN PATENT SPECIFICATION

(11) **EP 2 922 495 B1**
(45) Date of publication and mention of the grant of the patent: **03.05.2017**
(21) Application number: 13846247.8
(22) Date of filing: 20.11.2013
(51) Int. Cl.: A61F 2/01

(54) **VEIN FILTER**
VENENFILTER
FILTRE VEINEUX

(30) Priority: 20.11.2012 FR 1203119
(43) Date of publication of application: 30.09.2015
(73) Proprietor: B.Braun Medical SAS, 92100 Boulogne-Billancourt (FR)
(72) Inventor: GUTKNECHT, Dan, 63170 Pérignat lès Sarliève (FR); SENNEGON, Laurent, 86360 Chasseneuil-du-Poitou (FR)
(74) Representative: Winter, Brandl, Fürniss, Hübner, Röss, Kaiser, Polte - Partnerschaft mbB
(86) International application number: PCT/EP2013/003502
(87) International publication number: WO 2014/101978

(56) References cited:
- WO-A1-2009/088970
- WO-A1-2011/055079
- US-A1- 2004 158 273

## Description

This invention relates to a vein filter, which can switch from a collapsed state to a deployed state.

Vein filters are used for the prevention of pulmonary embolism. A collapsible filter is introduced into the vein through a catheter, usually in the inferior vena cava, to prevent blood clots travelling to the pulmonary artery. Removable filters are designed to be used temporarily, for example, during surgery, and then be removed.

The filters are collapsible so that they can be introduced into the inner lumen of a catheter and placed in a vein by release from the catheter. The distal end of the catheter is positioned at the desired location of the filter insertion and the latter is then released from the catheter. For this purpose, the filter is usually held in its position in the vein by a support device on its proximal end and then the catheter is removed gradually. The collapsed filter deploys out of the distal end of the catheter and is deployed into the vein.

A common type of filter is composed of metallic legs that open in an umbrella like manner, grouped on the proximal end at a filter head. In the collapsed state inside the catheter, the legs are under tension and are oriented approximately parallel to each other. Outside of the catheter, because of their elasticity, the legs move apart so that the filter can be deployed into the vein. Some legs have on their ends barbed hooks for anchoring the filter into the blood vessel walls.

Such a type of filter is described, for example, in the document US 2010/0318115 A1. However, there are limits to the application of such a vein filter given the necessary large cross-section width of the insertion or extraction catheter. Therefore, the smallest possible diameter of the filter in the collapsed state is desirable. However, a very small diameter in the collapsed state presents a risk of the hooks of the legs becoming entangled and thus, the filter not deploying properly when it is released from the catheter.

Furthermore, documents WO 2011/055079 A1, WO 2009/088970 A1 and US 2014/0158273 A1 exemplarily disclose vein filters that can switch from a collapsed state to a deployed state and that comprise legs with non-uniform lengths. At free ends of those legs hooks are provided for engaging with a respective blood vessel wall, when the vein filters are deployed inside of a blood vessel.

The goal of the present invention is to develop a vein filter concept that has a small diameter in the collapsed state and is easy to position without the risk of the legs becoming entangled when deploying the filter.

This goal is attained by a vein filter that can switch from a collapsed state to a deployed state and that comprises at least four legs extending from the filter head to a free end. Said four legs are organized in facing pairs, said facing pairs of legs having different lengths.

This leg configuration of the invention makes it possible to collapse the filter and insert it into the lumen of a catheter having a very small diameter. The free ends of the facing legs of each pair of legs collapse and deploy in opposite directions. The risk of the legs becoming entangled and not deploying properly is very low. The two pairs of legs have slightly different lengths so that the leg ends of one pair are slightly offset relative to the other pair. This reduces considerably the likelihood that legs of different pairs of legs hook on to each other.

Of course, the filter of the invention may comprise other legs in addition to the four legs described, which do not necessarily show the characteristics of the invention. For example, a filter may have four legs designed in accordance with the invention that are used for anchoring the filter and that have hooks on their free ends. The hooks may be directed outwards to be able to anchor in the blood vessel wall. Additional legs may have significantly different lengths and serve to stabilise the filter. Such a filter does not tilt due to the contact points between the legs and the vessel wall in different axial planes of the vessel. The filter is maintained upright in the vessel with the filter head in the axis of the vessel.

According to the present invention, the filter legs have a rectangular or oval shape in the cross section in the area of their free ends. The facing pairs of legs have thus a large bearing surface in the collapsed state. They are not likely to cross over and attach to each other when they are placed under tension in a catheter.

In addition, filter legs having a rectangular or oval cross section in the area of their free ends protect the vascular wall from injury, while providing reliable anchoring and positioning of the filter. A rectangular or oval shape in the cross section produces a tangential surface of the legs relative to the circumference of the filter. Once the filter is implanted, this area serves as contact area with the vessel wall. It can be sized such that the vessel wall is protected from injury, particularly by perforation.

According to a preferred embodiment of this invention, the hooks can be dimensioned and shaped so as not to protrude beyond the circumference of a circle having a diameter corresponding to the maximum width of a leg. The hooks can for example be of triangular shape. The filter legs are in their collapsed state almost straight, and the flat, rectangular cross section induces that the legs are positioned close to the middle plane of a catheter. Hooks shaped as described above will neither get into contact with the catheter wall nor hook up on to the catheter tip when the filter is introduced into the catheter or in case the filter will be retrieved from the vessel later on.

The legs may be slightly curved inwards before the hook turns outwards. The hooks will in this case even less likely hook on to the catheter tip when the filter is inserted into the catheter, and, in addition, the penetration depth of the hook in the vascular wall is limited and the vessel is not likely to be punctured. With this configuration, the shorter pair of legs can collapse inside the longer pair of legs. The dimensions of the collapsed filter are, therefore, particularly small.

If the legs had a rectangular or oval shape in the cross section along their whole length, this would result in a very large filter head diameter where all legs meet.

The cross section of the legs in an area close to the filter head are hence reduced and in particular circular. The legs can then be grouped in a small diameter filter head. The overall diameter of the filter in the collapsed state is reduced compared to conventional filters, ensuring at the same time strong anchoring in the vessel by virtue of the large contact surface in that area. It is thus possible to implant a filter having these characteristics in smaller catheters.

The cross section of the legs in the area close to the filter head may in particular allow the legs to twist. The result is that the legs may align their contact surface with the vessel wall to obtain a maximum contact surface and hence to protect the wall from injury.

Preferred embodiments of the present invention are described in more detail below with respect to the accompanying drawings:
**Fig. 1** shows a perspective view of the leg of a filter according to the invention;
**Fig. 2** shows a schematic representation of the free ends of two pairs of legs of a filter in accordance with the invention in the collapsed state;
**Fig. 3** shows a perspective view of the filter of the invention with a partial enlargement in figure 3a.

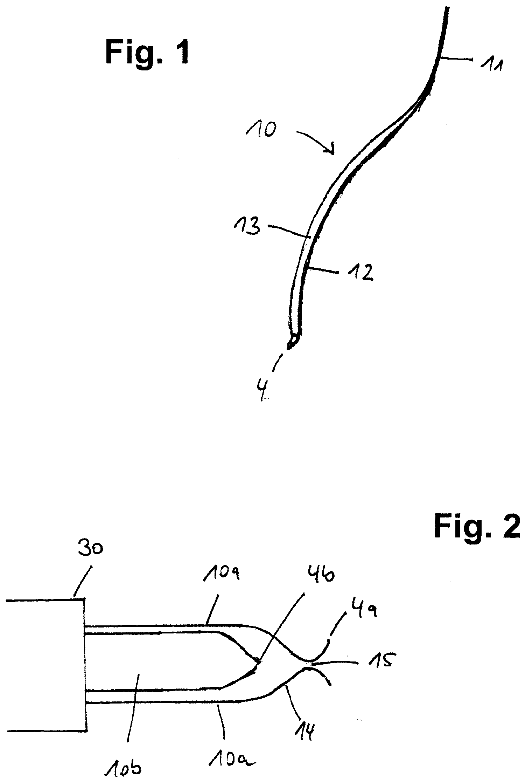

**Fig. 1** shows a leg **10** of a filter in accordance with the invention. The leg **10** has an area **11** close to the filter head and another area **12** near its free end,
which comes into contact with the vessel wall after installing the filter. The cross section of the area **12** is rectangular and the external surface **13** extends essentially tangentially to the circumference of the deployed filter. The cross section of the area **11** is circular.

The leg **10** extends from the filter head in a first section concavely to the outside and then bends convexly inwards in the area **12** above the hook **4**.

**Fig. 2** shows a schematic representation of the ends of the filter legs **10a**, **10b** of a filter in accordance with the invention in the collapsed state. The filter has two pairs of legs **10a, 10b**, which have slightly different lengths. In this representation it is located partially within a catheter **30**. The figure shows how the ends of the legs of each pair **10a, 10b** overlap in the collapsed state. For clarity, the other legs that the filter could include are not represented here.

The four legs **10a, 10b** are arranged respectively opposite one another in pairs and these pairs are offset relative to each other by 90°. The pairs have slightly different lengths. The inner surfaces **15** of the legs are in contact with each other due to the effect of the inward convex curvature in the area **12** above the hooks **4** of the legs **10a**, **10b** (fig. 1). The tips **4b** of the pair of shorter legs **10b** are located inside the pair of longer legs **10a**. The filter can be collapsed into a small volume.

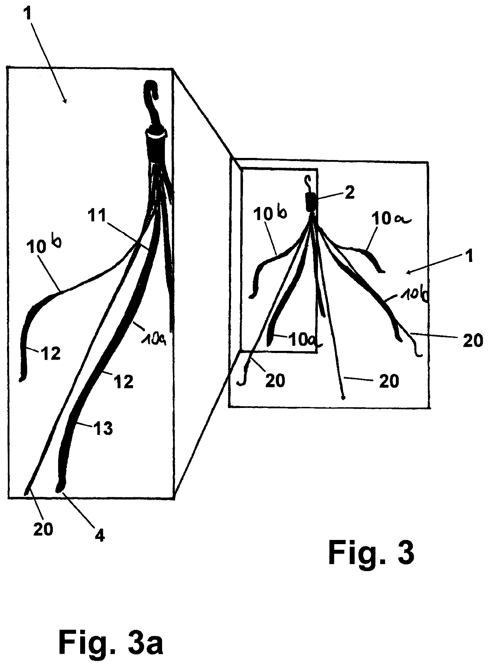

**Fig. 3** shows an embodiment of a filter **1** in accordance with the invention in the deployed state. Here, we must consider that the filter takes the form shown in the illustration, provided that it deploys without external limitations. The filter cannot be deployed to achieve this diameter in a cylindrical vessel, such as a vein. It is thus firmly anchored under slight tension in the vessel.

The filter **1** includes four legs **10a**, **10b** that extend respectively from a filter head **2** to a hooked free end **4.** The four legs **10a**, **10b** are arranged in two facing pairs, and the pairs of legs **10a**, **10b** have slightly different lengths. These legs **10a**, **10b** serve to anchor the filter in the vessel.

Besides the legs **10a**, **10b,** the filter has other legs **20** with significantly different lengths, which serve to stabilise the filter in a centred position in the vessel and avoid tilting of the filter once it is installed in the vessel.

The legs **10** have a circular diameter in the area **11** near the filter head **2.** Once the filter is inserted, this area does not come into contact with the vessel wall. In the area **12** distant from the filter head **2**, the legs **10** have a flat rectangular cross section, which produces a large surface **13**, extending essentially tangentially to the circumference of the deployed filter. Once the filter is in place, this surface area **13** is in contact with the vessel wall.

The flat surface **13** provides the legs **10a**, **10b** with a large contact surface against the vessel wall. Even with relatively high forces applied to the contact surface, there is no risk that the filter **1** damages or even perforates the vessel wall. However, with large bearing surfaces **13**, there is always the risk that the surface is not aligned with the vessel wall. In this case, there is a risk that the force that is applied to the vessel wall through an edge of the leg **10a**, **10b** leads to a risk of vascular wall trauma. The presently described embodiment of a filter according to the invention solves this problem by allowing the legs to twist in the cross section in the area **11** nearest the filter head. The rectangular area **12** of the filter automatically aligns with the vessel wall. The large surface area **13** is completely supported against the vessel wall.

The small circular cross section of the legs **10a**, **10b** in the area **11** near the filter head **2** also allows to save space when grouping the legs **10** at the filter head **2**. The diameter of the legs in the area **11** close to the filter head can be very small. The cross section changes when approaching the free ends of the filter to form a cross section with a tangentially extended outer surface **13** in the area **12**. This protects the vascular wall.

## Claims

1. A vein filter (1) that can switch from a collapsed state to a deployed state and comprising at least four legs (10a, 10b), which extend respectively from a filter head (2) to a free end, wherein
said four legs (10) are organised in facing pairs (10a, 10b),
said pairs of legs (10a, 10b) have different lengths, and
the cross sections of the legs (10) show a circular shape in the area (11) close to the filter head (2),
**characterised in that**
the cross sections of the legs (10) show a rectangular or oval shape in the area (12) of the free end.

2. A vein filter (1) according to claim 1, **characterised in that** the legs (10) have hooks (4) on their free ends.

3. A vein filter (1) according to claim 2, **characterised in that** the hooks (4) are triangular-shaped and directed outwards.

4. A vein filter (1) according to any of claims 1 to 3, **characterised in that** the legs (10) are curved inwards in a convex shape in the area (12) above the free end.

## Patentansprüche

1. Venenfilter (1), der von einem zusammengeklappten in einen aufgespannten Zustand wechseln kann und der zumindest vier Streben (10a, 10b) aufweist, die sich von einem Filterkopf (2) aus jeweils zu einem freien Ende hin erstrecken, wobei
die vier Streben (10a, 10b) in einander gegenüberliegenden Paaren (10a, 10b) gegliedert sind,
die Strebenpaare (10a, 10b) verschiedene Längen aufweisen, und
die Querschnitte der Streben (10) im Bereich (11) nahe des Filterkopfes (2) eine kreisrunde Form aufweisen,
**dadurch gekennzeichnet, dass**
die Querschnitte der Streben (10) im Bereich (12) des freien Endes eine rechteckige oder ovale Form aufweisen.

2. Venenfilter (1) gemäß Anspruch 1, **dadurch gekennzeichnet, dass** die Streben (10) an ihren freien Enden Haken (4) aufweisen.

3. Venenfilter (1) gemäß Anspruch 2, **dadurch gekennzeichnet, dass** die Haken (4) dreiecksförmig sind und nach außen weisen.

4. Venenfilter (1) gemäß einem der Ansprüche 1 bis 3, **dadurch gekennzeichnet, dass** die Streben (10) im Bereich 12 vor den freien Enden in einer konvexen Form nach innen gebogen sind.

## Revendications

1. Filtre veineux (1) qui peut commuter d'un état replié à un état déployé et comprenant au moins quatre pieds (10a, 10b), qui s'étendent respectivement depuis une tête de filtre (2) jusqu'à une extrémité libre, dans lequel
lesdits quatre pieds (10) sont organisés en paires se faisant face (10a, 10b),
lesdites paires de pieds (10a, 10b) ont des longueurs différentes, et
les sections transversales des pieds (10) présentent une forme circulaire dans la zone (11) proche de la tête de filtre (2),
**caractérisé en ce que**
les sections transversales des pieds (10) présentent une forme rectangulaire ou ovale dans la zone (12) de l'extrémité libre.

2. Filtre veineux (1) selon la revendication 1, **caractérisé en ce que** les pieds (10) ont des crochets (4) sur leurs extrémités libres.

3. Filtre veineux (1) selon la revendication 2, **caractérisé en ce que** les crochets (4) ont une forme triangulaire et sont dirigés vers l'extérieur.

4. Filtre veineux (1) selon l'une quelconque des revendications 1 à 3, **caractérisé en ce que** les pieds (10) sont incurvés vers l'intérieur en une forme convexe dans la zone (12) au-dessus de l'extrémité libre.
